# EUROPEAN PATENT SPECIFICATION

(11) **EP 0 475 628 B1**
(45) Date of publication and mention of the grant of the patent: **01.02.1995**
(21) Application number: 91307840.8
(22) Date of filing: 28.08.1991
(51) Int. Cl.: C07C 69/54, C07C 67/08, C08F 20/20

(54) **Acrylate esters of 1,1,1-trishydroxyphenylethane**
1,1,1-Trishdroxyphenyläthanacrylate
Acrylates du 1,1,1-trishydroxyphényléthane

(30) Priority: 31.08.1990 US 576630; 11.10.1990 US 595887
(43) Date of publication of application: 18.03.1992
(73) Proprietor: HOECHST CELANESE CORPORATION, Somerville, N.J. 08876 (US)
(72) Inventor: Mott, Graham N., Texas 78413 (US); Johnson, Thomas S., W-6240 Konigstein (DE)
(74) Representative: De Minvielle-Devaux, Ian Benedict Peter

(56) References cited:
- EP-A- 0 251 724
- EP-A- 0 322 166

## Description

The present invention relates to acrylate esters of 1,1,1-trishydroxyphenylethane (THPE) or more particularly to mono-, di-, and triacrylated esters of THPE. The invention also relates to a process for their preparation, their use as multifunctional polymerizable monomers, and homo- and copolymers produced therefrom. Such compounds find use in the production of protective coating compositions, adhesives, photosensitive compositions, and the like.

### Background of the invention

Acrylate esters of 1,1,1-trishydroxyphenylethane may be produced by reacting 1,1,1-tris(4′-hydroxyphenyl) ethane with an acrylating agent as hereinafter described. It is known in the art that the intermediate 1,1,1-tris(4′-hydroxyphenyl) ethane may be produced by reacting 4-hydroxyacetophenone with phenol. Typically this is performed with phenol also used as the solvent for the mixture. THPE itself may be produced according to a method described in U.S. patent application 07/478,072, filed February 9, 1990, and European patent application EP-A-0 441 648, filed 8 February 1991. According to that disclosure, very pure THPE is obtained by washing a crude reaction mixture of 4-hydroxyacetophenone and phenol with a THPE saturated wash/methanol liquid prior to a recrystallization. It has been found that by use of such a wash yields of pure white THPE are good, and solvents have a more productive use. As a result of the process, most of the color bodies are removed in the wash stage where the color of the crude THPE changes from a dark rusty color to a light tan. The balance is removed in the recrystallization. With crude THPE washed with recycled recrystallization filtrate, only a single recrystallization from methanol/water is necessary to meet white color and high purity requirements. This recrystallization is basically a precipitation of THPE from the methanol/water solution. By acrylating THPE, one produces the inventive monomeric mono-, di- or tri-acrylate esters of 1,1,1-trishydroxyphenylethane. These may be polymerized, copolymerized and/or crosslinked by the use of known free radical polymerization and crosslinking techniques.

### Summary of the Invention

The invention provides, as a composition of matter, mono-, di- and tri-acrylate esters of 1,1,1-trishydroxyphenylethane. The invention also provides a method for the production of acrylate esters of 1,1,1-trishydroxyphenylethane which comprises acrylating 1,1,1-tris(4′-hydroxyphenyl)ethane. The inventive compounds have the general formula 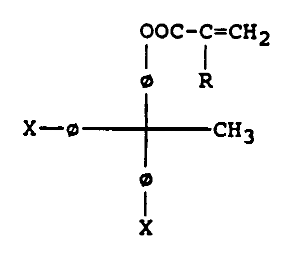
where φ = a phenylene group
X = -OH or 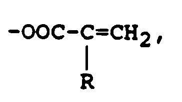
and
R = H, alkyl or aryl, preferably C₁-C₆ alkyl or C₆-C₁₀ aryl.

The invention also comprises homopolymers and copolymers of these monomers as well as a method for preparing such homopolymers and copolymers by free radical initiating the monomers. Articles may be prepared by disposing such compositions on substrates such as metals, plastics, and the like.

### Detailed Description of the Preferred Embodiment

As hereinbefore mentioned, the production of 1,1,1-tris(4′-hydroxyphenyl)ethane may be performed by the reaction of 4-hydroxyacetophenone with phenol, wherein phenol is the supporting solvent as well as a reagent. The reaction takes place under catalytic conditions, with hydrochloric acid and beta-mercaptopropionic acid as preferred co-catalyst. The resulting reaction product contains significant amounts of impurities which are removed as described hereinafter. An impure, substantially solid crude admixture of freshly produced THPE contains 1,1,1-tris(4′-hydroxyphenyl)ethane (THPE), residual 4-hydroxyacetophenone, phenol, chlorides, THPE isomers, bis-(hydroxyphenyl)ethene isomers, color bodies and other unidentified parts which are sought to be removed. 1,1,1-Tris(4′-hydroxyphenyl)ethane may be obtained from a substantially solid crude admixture containing 1,1,1-tris(4′-hydroxyphenyl)ethane and the impurities resulting from the catalytic production of 1,1,1-tris(4′-hydroxyphenyl)ethane from 4-hydroxyacetophenone and phenol. The THPE purification method proceeds by:
a) washing the crude admixture with a saturated solution of 1,1,1-tris(4′-hydroxyphenyl)ethane in a solute comprising from 60% to 75% by weight of water and from 25% to 40% by weight of methanol; and
b) isolating the washed crude admixture from the formed effluent washing composition, and dissolving the washed crude admixture in methanol, and
c) adding sufficient water and sodium borohydride to the dissolved, washed crude admixture to form a precipitate of 1,1,1-tris(4′-hydroxyphenyl)ethane, and
d) filtering the precipitate to thereby form a purified 1,1,1-tris(4′-hydroxyphenyl)ethane and a filtrate; and
e) rinsing the resultant filtered precipitate of 1,1,1-tris(4′-hydroxyphenyl)ethane with a solution of sufficient methanol and water, which optionally contains 1,1,1-tris(4′-hydroxyphenyl)ethane up to the saturation point, and conducting the rinsing for a sufficient time to remove substantially all residual colored impurities from the precipitate.

In the first step of the purification method, one washes the crude admixture with a saturated solution of 1,1,1-tris(4′-hydroxyphenyl)ethane in a solute comprising from 60% to 75% by weight of water and from 25% to 40% by weight of methanol. Preferably this washing is conducted in several washing steps. It has been found that by employing a saturated solution of THPE in the washing solution, that THPE loss from the crude admixture is substantially reduced. Prior to washing, the crude admixture typically contains from 15% to 30% by weight of residual phenol. Since-phenol is a good solvent for THPE, it is desired to reduce the phenol content prior to the washing step simply by vacuum draw of the phenol.

However, it has been found that if too much phenol is drawn off, that although the THPE recovery is good, the purity of the product is unsatisfactory. Loss of THPE during the wash of the crude admixture is basically caused by the presence of phenol although the washing solution is THPE saturated. Phenol content may be controlled by pumping off the crude admixture before the wash or by use of a hot nitrogen flow through the crude admixture to cause the phenol, which is the most volatile component, to leave the system before the wash. Generally it is desired to obtain THPE with a 99.5% or greater purity and having a whiteness measure (APHA) of 200 or less, preferably 150 or less. Therefore a phenol content of at least 1.0% and up to 30.0% based on the weight of the crude admixture is desired. More preferably the phenol content is adjusted to from 4.0% to 10% and most preferably from 4.5% to 7.5%. A single most preferred phenol content is 5.0%. Therefore, in the most preferred case, the phenol content of the crude admixture is first adjusted to these levels before conducting the washing step.

Next, after the washing step, one isolates the washed crude admixture from the formed effluent washing composition. This can be done by performing the washing on a filter plate while stirring and then drawing down the filtrate. The solid, washed crude admixture is then dissolved in sufficient methanol to effect a dissolution.

One then adds sufficient water and sodium borohydride to reduce the dissolved, washed crude admixture and to form a precipitate of 1,1,1-tris(4′-hydroxyphenyl)ethane. The sodium borohydride also acts as a pH adjusting reagent during the recrystallization. In the preferred embodiment, the amount of sodium borohydride added ranges from 0.0003% to 0.3%, preferably from 0.003% to 0.07% and most preferably from 0.01% to 0.03% based on the weight of methanol and water. If the phenol content in the crude admixture has been reduced prior to washing, it is preferred that sodium borohydride is added to the methanolic solution THPE solution. After stirring, carbon in the form of charcoal, is added to the methanolic THPE solution and filtered off prior to having added more sodium borohydride plus water. This is most advantageous when the phenol content has been reduced to 15% or less in the pre-washed crude admixture. In the preferred embodiment, the amount of carbon added ranges from 0.001% to 1.0%, preferably from 0.01% to 0.8%, and most preferably from 0.05% to 0.3% based on the weight of methanol.

One then filters the precipitate to thereby form a purified, recrystallized 1,1,1-tris(4′-hydroxyphenyl)ethane and a filtrate. The next step is rinsing the resultant filtered precipitate of 1,1,1-tris(4′-hydroxyphenyl)ethane with a solution of sufficient methanol and water, which optionally contains 1,1,1-tris(4′-hydroxyphenyl)ethane up to the saturation point, and conducting the rinsing for a sufficient time to remove substantially all residual colored impurities from said precipitate. The rinse mixture preferably comprises water and methanol in a 2:1 to 6:1 weight ratio. One may perform an optional stabilizing rinse with an aqueous sodium dithionate solution at this stage. Typical sodium dithionate solutions may range from 0.01% to 1.0%, preferably from 0.05% to 0.5% by weight in water. Finally, the product is dried.

It has been found that one may use this colored, resultant filtrate as the washing solution in the washing step of another crude admixture batch. Although this filtrate contains sodium borohydride and removed, colored bodies and other impurities in addition to water, methanol and THPE, it has been found that these are substantially removed in the effluent of the washing step. If the filtrate color is pink one should treat it with sodium dithionate to change it to a light yellow color prior to using the filtrate to wash the next batch of crude admixture. A suitable amount is about 0.1 weight percent of saturated aqueous sodium dithionate. Therefore, this filtrate serves double duty in the overall process and significantly reduces the amount of fluids which must be recycled or treated before ultimate discharge.

The acrylate esters of 1,1,1-trishydroxyphenylethane may be produced by reacting the 1,1,1-tris(4′-hydroxyphenyl)ethane with an acrylating agent under suitable reaction conditions. The degree of esterification may be controlled by regulating the amount of the acrylating agent used. The most preferred acrylating agent is acryloyl chloride.

The monomer may be polymerized by a free radical initiation process to produce homopolymers such that it has a molecular weight in the range of from 200,000 to 10,000,000 preferably from 200,000 to 1,000,000 or more preferably 500,000 to 1,000,000. It is predicted that essentially any free radical initiation system will serve for this purpose. One free radical initiation method is by photopolymerization wherein the acrylated THPE is admixed with a photopolymerization initiator in a suitable solvent composition and thereafter exposed to light. One preferred free radical initiator is azoisobutyronitrile. Other azo type initiators are also suitable. Still others non-exclusively include peroxides such as benzoyl peroxide, and di-t-butyl peroxide. Free radical liberating photoinitiators include any compound which liberates free radicals on stimulation by actinic radiation. Preferred photoinitiators nonexclusively include quinoxaline compounds as described in U. S. Patent 3,765,898; the vicinal polyketaldonyl compounds in U. S. Patent 2,367,660; the alpha-carbonyls in U.S. Patents 2,367,661 and 2,367,670; the acyloin ethers in U. S. Patent 2,448,828; the triarylimidazolyl dimers in U. S. Patent 3,479,185; the alpha-hydrocarbon substituted aromatic acyloins in U. S. Patent 2,722,512; polynuclear quinones in U. S. Patents 2,951,758 and 3,046,127; and s-triazines in U. S. Patent 4,656,272. In the practice of the present invention, the photoinitiator component is preferably blended with the acrylated THPE, in a suitable solvent composition, an amount ranging from approximately 2% to 30% based on the weight of the solids in the mixture. A more preferred range is from approximately 6% to 20%.

The polymer set forth above is preferably a homopolymer, but it may also be a copolymer wherein the co-monomers are units of styrene, acrylates, methacrylates and maleimides. The co-monomer unit may optionally be substituted with a variety of pendant groups in order to adjust the properties of the compound as desired by the user. Specifically, the aforementioned acrylated THPE monomer may be co- or terpolymerized under the foregoing polymerization conditions in the presence of one or more additional monomers which may be ethylenically unsaturated compounds, acetoxystyrene, substituted acetoxystyrene, hydroxystyrene, substituted hydroxystyrene, maleic anhydride, maleimides, styrene, acrylates and methacrylates such as methyl methacrylate, and butyl acrylate wherein the copolymer or terpolymer has an average molecular weight in the range given above.

Preferably the acrylated THPE monomer is co-polymerized with a compound which is an addition polymerizable, nongaseous (boiling temperature above 100°C at normal atmospheric pressure), ethylenically unsaturated compound containing at least two terminal ethylenically unsaturated groups, which is capable of forming a high molecular weight polymer by free radical initiated, chain propagating addition polymerization. Suitable polymerizable materials nonexclusively include triethylene glycol dimethacrylate, tripropylene glycol diacrylate, tetraethylene glycol dimethacrylate, diethylene glycol dimethacrylate, 1,4-butanediol diacrylate, 1,6-hexanediol dimethacrylate, pentaerythritol tetraacrylate, trimethylol propane triacrylate, trimethylol propane trimethacrylate, di-pentaerythritol monohydroxypentaacrylate, pentaerythritol triacrylate, bisphenol A ethoxylate dimethacrylate, trimethylolpropane ethoxylate triacrylate, and trimethylolpropane propoxylate triacrylate.

One may employ the foregoing homopolymers, copolymers and terpolymers in protective surface coatings and adhesive blends. They may also be used in admixture with a photosensitizing agent to form a photographic element such as a photoresist. In the production of a photosensitive composition and photographic element, one blends the above produced polymer with a photosensitizer and a suitable solvent until a homogeneous solution is formed. The solution is then coated on a suitable substrate and dried until it is non-tacky. It is then imagewise exposed to light and developed. Photosensitizers are known to the skilled artisan as demonstrated by Light Sensitive Systems, Kosar, J.; John Wiley & Sons, New York, 1965. The monomer may also be used as a component of a photopolymerizable element wherein the monomer, initiator and a solvent are coated on a support, dried, and imagewise exposed to light with subsequent development. In another embodiment, the acrylated polymer may be crosslinked by admixture with a crosslinking agent with subsequent heating. In one case the acrylated THPE polymer and crosslinking agent are mixed, coated on a support, and crosslinked with heat in the presence of a catalytic amount of an acid.

The crosslinking component is a compound, which when in the presence of heat, and preferably a catalytic amount of acid, is capable of crosslinking the foregoing acrylated THPE polymer. Crosslinking compounds for such use have the general formula

R′O-CH₂-A-CH₂-OR′

wherein A is a monomeric, aromatic hydrocarbon having one or more fused or unfused, separated or unseparated rings and each R unit is independently H, (C₁-C₆)alkyl, (C₃-C₆) cycloalkyl, aryl or arylalkyl.

The preferred crosslinking compound is dimethylol paracresol as described in U.S. 4,404,272, and its ether and ester derivatives including phenol, 2,6-bis(hydroxyethyl)-4-methyl; benzene, 1-methoxy-2,6-bis-(hydroxymethyl-4-methyl; phenol, 2,6-bis(methoxymethyl)-4-methyl; and benzene, 1-methoxy-2,6-bis(methoxymethyl)-4-methyl; methyl methoxy diphenyl ether, melamine formaldehyde resins and compounds and alkylated analogous thereof having 1 to about 3 monomer units such as those typically sold under the trade names Cymel from American Cyanamid and Resimene from the Monsanto Company, for example hexamethylol melamine hexamethyl ether.

In the preferred photoresist composition, the crosslinking compound is present in a mixture with the acrylated THPE monomer, polymer or copolymer in an amount of from 0.5 to 7 weight percent of the solid components of the composition, more preferably from 2 to 6 weight percent, and most preferably from 3 to 5 weight percent.

The following non-limiting examples serve to illustrate the invention.

### Example 1

### 1,1,1,-tris(4′-acryloxyphenyl)ethane

318 g (1.04 mol) 1,1,1-Trishydroxyphenylethane, 314 g (3.08 mol) triethylamine, 0.75 g methylhydroquinone and 500 ml of methylene chloride are added to a 5 liter flask and cooled to 0°C. A mixture of 300 g (3.3 mol) of acryloyl chloride, and 1300 ml of methylene chloride are added over a four hour period while maintaining the temperature at 0°C. When the mixing is complete, the mixture is allowed to warm to room temperature and stirred overnight. Several liters of water are added to precipitate triethylammonium hydrochloride. The organic phase is separated and dried over magnesium sulfate. After adding an additional 0.25 grams of methylhydroquinone, the organic phase is evaporated using an air sparge, affording a light yellow viscous liquid.

### Example 2

### 1,1,1-tris(4′-acryloxyphenyl)ethane

30.6 g (0.1 mol) of 1,1,1-trishydroxyphenylethane, 30.3 g (0.3 mol) of triethylamine, 0.25 grams of methylhydroquinone and 150 ml of tetrahydrofuran are added to a flask at 0°C. A mixture of 27.5 g (0.3 mol) of acryloyl chloride and 50 ml of tetrahydrofuran are added over three hours and left to stir overnight. 200-300 ml of water are added. The tetrahydrofuran solution is precipitated, evaporated and dried to yield 48-49 grams of a thick, light yellow liquid.

### Example 3

111.3 grams of 1,1,1-trishydroxyphenylethane, 110.2 grams of triethylamine, 0.25 grams of methylhydroquinone and 200 ml of methylene chloride are added to a flask at 0°C. Then 100 grams of acryloyl chloride, and 500 ml of methylene chloride are added over four hours to yield a light yellow solution with a heavy precipitate. Two liters of water are added. The methylene chloride is extracted and the mixture is dried. 0.25 grams of methylhydroquinone are added and the mixture is evaporated under an oxygen sparge.

## Claims

1. Mono-, di- and tri- acrylate esters of 1,1,1-trishydroxyphenylethane having the formula 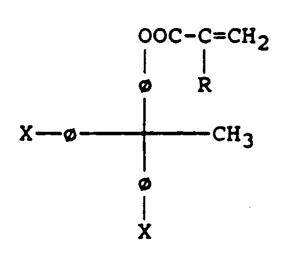 where
φ = a phenylene group
X = -OH or 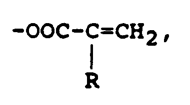 and
R = H, alkyl or aryl.

2. The acrylated esters of claim 1 wherein R is C₁-C₆ alkyl or C₆-C₁₀ aryl.

3. The acrylated esters of claim 1 wherein each X is

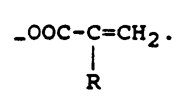

4. A composition which comprises in admixture
(a) a mono-, di- and tri- acrylate esters of 1,1,1-trishydroxyphenylethane having the formula 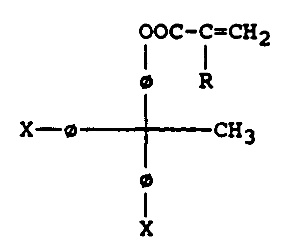 where
φ = a phenylene group
X = -OH or 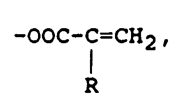 and
R = H, alkyl or aryl
(b) one or more compounds selected from the group consisting of free radical polymerization initiators and crosslinking agents.

5. An article which comprises the composition of claim 4 disposed upon a substrate.

6. The composition of claim 4 comprising a free radical polymerization initiator selected from the group consisting of an azo compound, a peroxide, quinoxaline compounds, polyketaldonyl compounds, alpha-carbonyl compounds, acyloin ethers, triarylimidazolyl dimers, alpha-hydrocarbon substituted aromatic acyloins, polynuclear quinones, and s-triazines.

7. The composition of claim 4 comprising a crosslinking compound having the general formula
R′O-CH₂-A-CH₂-OR′
wherein A is a monomeric, aromatic hydrocarbon having one or more fused or unfused, separated or unseparated rings and each R′ unit is independently H, (C₁-C₆)alkyl, (C₃-C₆) cycloalkyl, aryl or arylalkyl.

8. The composition of claim 4 wherein R is C₁-C₆ alkyl or C₆-C₁₀ aryl.

9. The composition of claim 4 wherein each X is

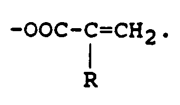

10. A polymer or copolymer of the formula 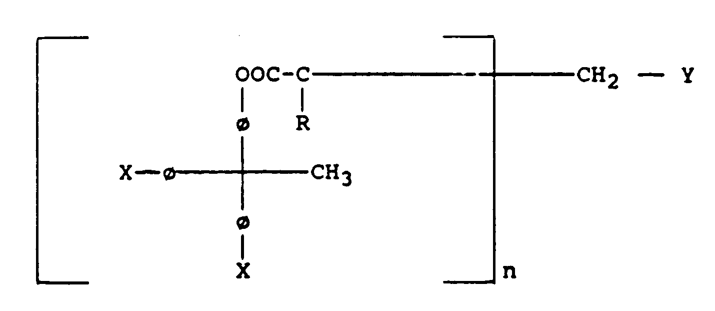 Where
φ = a phenylene group
X = -OH, 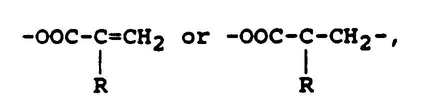 R = H, alkyl or aryl
Y is selected from the group consisting of a residue of 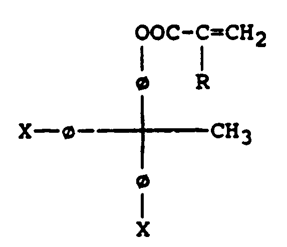 and the residue of an ethylenically unsaturated compound; and n is selected to yield a polymer having a molecular weight in the range of from 200,000 to 10,000,000.

11. The polymer of claim 10 wherein R is C₁-C₆ alkyl or C₆-C₁₀ aryl.

12. The polymer of claim 10 wherein each X is

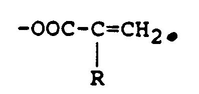

13. The polymer of claim 10 wherein Y is a residue of

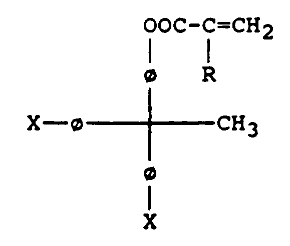

14. The polymer of claim 13 wherein R is C₁-C₆ alkyl or C₆-C₁₀ aryl.

15. The polymer of claim 13 wherein each X is

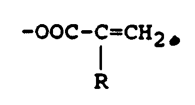

16. A copolymer according to claim 10 wherein Y is selected from the group consisting of a residue of acetoxystyrene, hydroxystyrene, maleic anhydride, maleimides, styrene, acrylates and methacrylates.

17. A composition comprising the polymer or copolymer of claim 10 in admixture with a crosslinking compound.

18. The composition of claim 17 wherein the crosslinking compound has the formula
R′O-CH₂-A-CH₂-OR′
wherein A is a monomeric, aromatic hydrocarbon having one or more fused or unfused, separated or unseparated rings and each R′ unit is independently H, (C₁-C₆)alkyl, (C₃-C₆) cycloalkyl, aryl or arylalkyl.

19. An article which comprises the composition of claim 17 disposed upon a substrate.

20. A method for the production of mono-, di- and tri-acrylate esters of 1,1,1-trishydroxyphenylethane having the formula 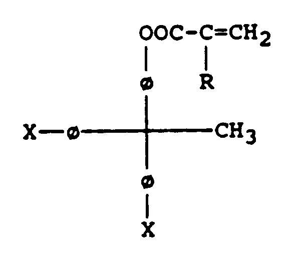 where
φ = a phenylene group
X = -OH or 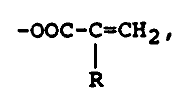 and
R = H, alkyl or aryl which comprises reacting about one equivalent of 1,1,1-tris(4′-hydroxyphenyl)ethane with from about 1 to about 3 equivalents of an acrylating agent in an amount sufficient and for a sufficient time, and at a sufficient temperature to produce an acrylate ester of 1,1,1-trishydroxyhenylethane.

21. The method of claim 20 wherein R is C₁-C₆ alkyl or C₆-C₁₀ aryl.

22. The method of claim 21 wherein each X is

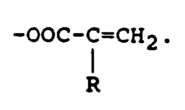

23. The method of claim 21 wherein the acrylating agent is acryloyl chloride.

24. A method of producing a polymer or copolymer of the formula 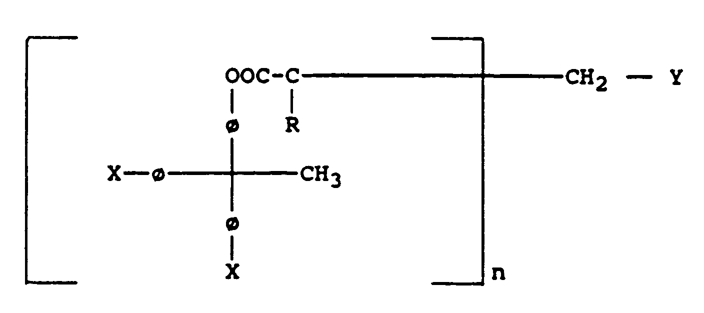 which comprises free radical polymerizing a compound having the formula 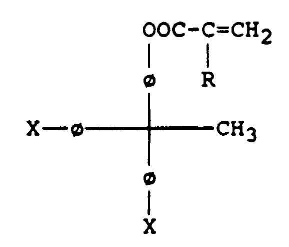 with a compound Y which is selected from the group consisting of a residue of 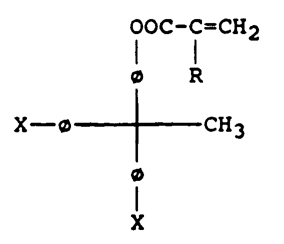 and the residue of an ethylenically unsaturated compound;
where
φ = a phenylene group
X = -OH, 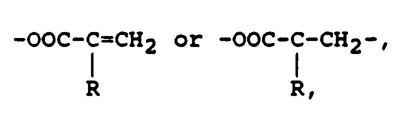 and
R = H, alkyl or aryl; and
n is selected to yield a polymer having a molecular weight in the range of from 200,000 to 10,000,000.

25. The method of claim 24 which is conducted with a free radical polymerization initiator selected from the group consisting of an azo compound, a peroxide, quinoxaline compounds, polyketaldonyl compounds, alpha-carbonyl compounds, acyloin ethers, triarylimidazolyl dimers, alpha-hydrocarbon substituted aromatic acyloins, polynuclear quinones, and s-triazines.

## Patentansprüche

1. Mono-, Di- und Triacrylatester von 1,1,1-Trishydroxyphenylethan mit der Formel 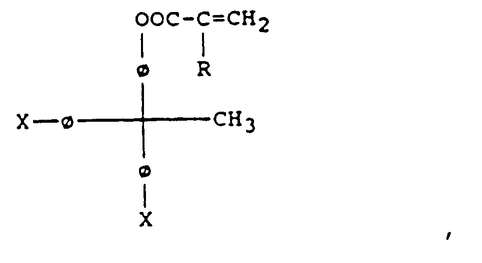 wobei
φ = eine Phenylengruppe,
X = -OH oder 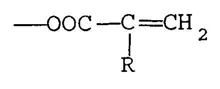 und
R = H, Alkyl oder Aryl ist.

2. Acrylierte Ester nach Anspruch 1, wobei R ein C₁-C₆-Alkyl oder ein C₆-C₁₀-Aryl ist.

3. Acrylierte Ester nach Anspruch 1, wobei jedes X 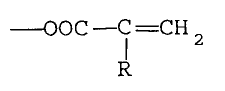 ist.

4. Zusammensetzung, umfassend als Mischung
(a) Mono-, Di- und Triacrylatester von 1,1,1-Trishydroxyphenylethan mit der Formel 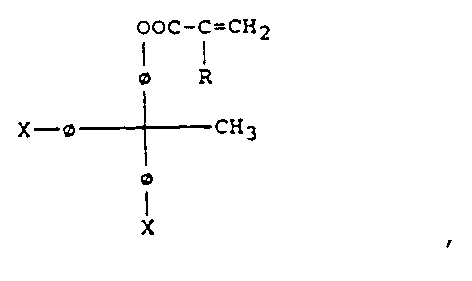 wobei
φ = eine Phenylengruppe,
X = -OH oder 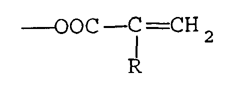 und
R = H, Alkyl oder Aryl ist.
(b) eine oder mehrere Verbindungen, ausgewählt aus der aus radikalischen Polymerisations-Initiatoren und Vernetzungsmitteln bestehenden Gruppe.

5. Gegenstand, der die auf einem Substrat abgeschiedene Zusammensetzung nach Anspruch 4 umfaßt.

6. Zusammensetzung nach Anspruch 4, umfassend einen radikalischen Polymerisations-Initiator, ausgewählt aus der aus einer Azoverbindung, einem Peroxid, Chinoxalin-Verbindungen, Polyketaldonyl-Verbindungen, Alpha-Carbonyl-Verbindungen, Acyloinethern, Triarylimidazolyldimeren, durch Alpha-Kohlenwasserstoffe substituierten aromatischen Acyloinen, mehrkernigen Chinonen und s-Triazinen bestehenden Gruppe.

7. Zusammensetzung nach Anspruch 4, umfassend eine vernetzende Verbindung mit der allgemeinen Formel
R'O-CH₂-A-CH₂-OR',
worin A ein monomerer, aromatischer Kohlenwasserstoff mit einem oder mehreren, kondensierten oder nicht kondensierten, getrennten oder nicht getrennten Ringen ist und jede Einheit R' unabhängig voneinander H, (C₁-C₆)-Alkyl, (C₃-C₆)-Cycloalkyl, Aryl oder Arylalkyl ist.

8. Zusammensetzung nach Anspruch 4, wobei R ein C₁-C₆-Alkyl oder ein C₆-C₁₀-Aryl ist.

9. Zusammensetzung nach Anspruch 4, wobei jedes X 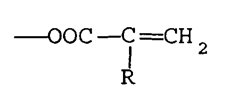 ist.

10. Polymer oder Copolymer mit der Formel 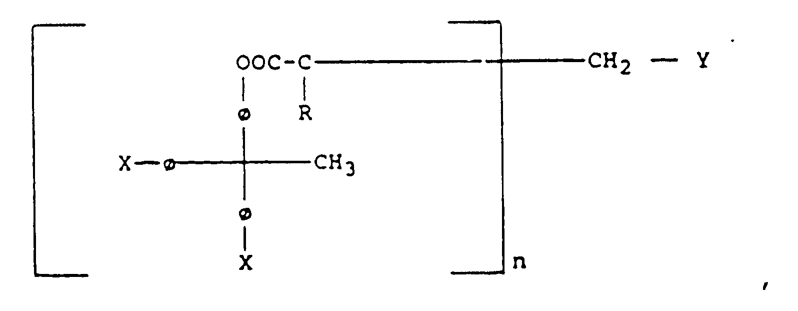 wobei
φ = eine Phenylengruppe,
X = -OH, 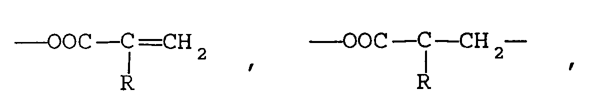 R = H, Alkyl oder Aryl,
Y aus der aus einem Rest 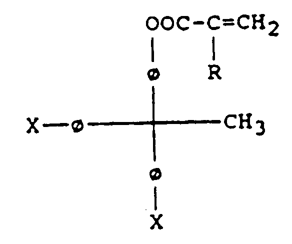 und dem Rest einer ethylenisch ungesättigten Verbindung bestehenden Gruppe ausgewählt ist und n so ausgewählt ist, daß sich ein Polymer mit einem Molekulargewicht im Bereich von 200 000 bis 10 000 000 ergibt.

11. Polymer nach Anspruch 10, wobei R ein C₁-C₆-Alkyl oder ein C₆-C₁₀-Aryl ist.

12. Polymer nach Anspruch 10, wobei jedes X 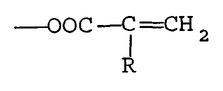 ist.

13. Polymer nach Anspruch 10, wobei Y ein Rest 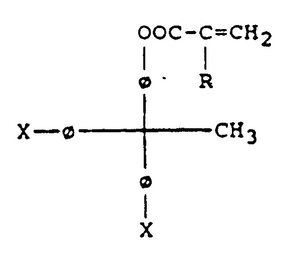 ist.

14. Polymer nach Anspruch 13, wobei R ein C₁-C₆-Alkyl oder ein C₆-C₁₀-Aryl ist.

15. Polymer nach Anspruch 13, wobei jedes X 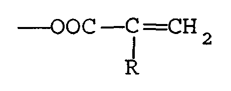 ist.

16. Copolymer nach Anspruch 10, wobei Y aus der aus einem Rest von Acetoxystyrol, Hydroxystyrol, Maleinsäureanhydrid, Maleinsäureimiden, Styrol, Acrylaten und Methacrylaten bestehenden Gruppe ausgewählt ist.

17. Zusammensetzung, umfassend das Polymer oder Copolymer nach Anspruch 10 in Abmischung mit einer vernetzenden Verbindung.

18. Zusammensetzung nach Anspruch 17, wobei die vernetzende Verbindung die Formel
R'O-CH₂-A-CH₂-OR'
aufweist, wobei A ein monomerer, aromatischer Kohlenwasserstoff mit einem oder mehreren kondensierten oder nicht kondensierten, getrennten oder nicht getrennten Ringen ist und jede Einheit R' unabhängig voneinander H, (C₁-C₆)-Alkyl, (C₃-C₆)-Cycloalkyl, Aryl oder Arylalkyl ist.

19. Gegenstand, der die auf einem Substrat abgeschiedene Zusammensetzung nach Anspruch 17 umfaßt.

20. Verfahren zur Herstellung von Mono-, Di- und Triacrylatestern von 1,1,1-Trishydroxyphenylethan mit der Formel 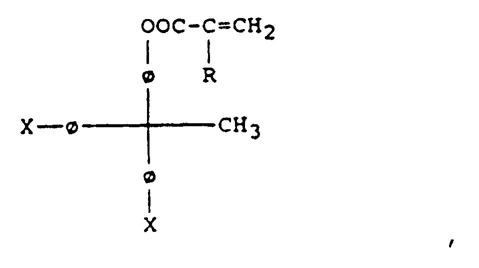 wobei
φ eine Phenylengruppe,
X = -OH oder 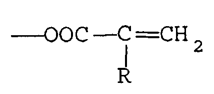 und
R = H, Alkyl oder Aryl ist,
die das Umsetzen ungefähr eines Equivalents von 1,1,1-Tris(4'-hydroxyphenyl)ethan mit ungefähr 1 bis ungefähr 3 Equivalenten eines acrylierenden Mittels in einer ausreichenden Menge und für einen ausreichenden Zeitraum und bei einer Temperatur, die ausreichend ist, um einen Acrylatester von 1,1,1-Trishydroxyphenylethan herzustellen, umfaßt.

21. Verfahren nach Anspruch 20, wobei R ein C₁-C₆-Alkyl oder ein C₆-C₁₀-Aryl ist.

22. Verfahren nach Anspruch 21, wobei jedes X 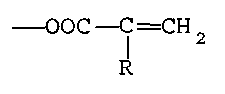 ist.

23. Verfahren nach Anspruch 21, wobei Acryloylchlorid das acrylierende Mittel ist.

24. Verfahren zur Herstellung eines Polymers oder Copolymers mit der Formel 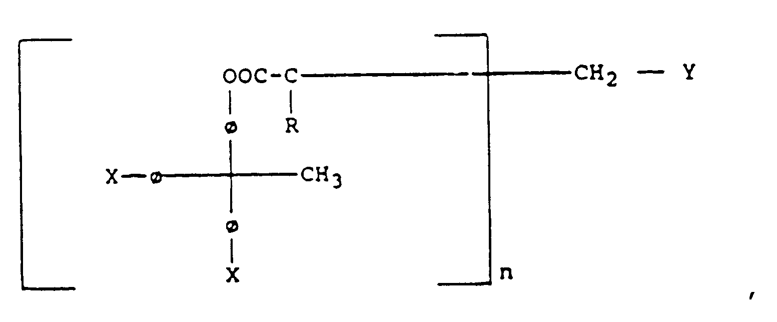 das die radikalische Polymerisation einer Verbindung mit der Formel 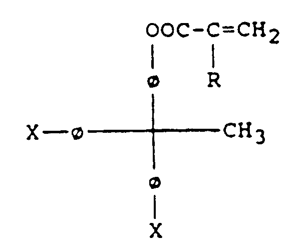 mit einer Verbindung Y umfaßt, die aus der aus einem Rest von 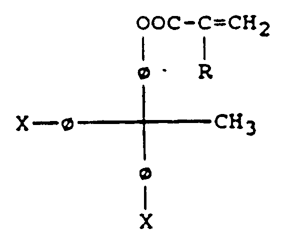 und dem Rest einer ethylenisch ungesättigten Verbindung ausgewählt ist, wobei φ = eine Phenylengruppe,
X = -OH, 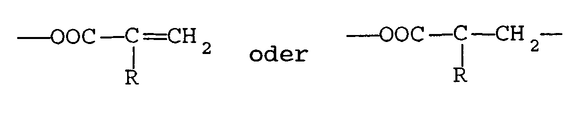 und R = H, Alkyl oder Aryl und
n so ausgewählt ist, daß sich ein Polymer mit einem Molekulargewicht im Bereich von 200 000 bis 10 000 000 ergibt.

25. Verfahren nach Anspruch 24, das mit einem radikalischen Polymerisations-Initiator durchgeführt wird, der aus der aus einer Azoverbindung, einem Peroxid, Chinoxalin-Verbindungen, Polyketaldonyl-Verbindungen, Alpha-Carbonyl-Verbindungen, Acyloinethern, Triarylimidazolyldimeren, durch Alpha-Kohlenwasserstoffe substituierten aromatischen Acyloinen, mehrkernigen Chinonen und s-Triazinen bestehenden Gruppe ausgewählt ist.

## Revendications

1. Esters mono-, di- et tri-acryliques de 1,1,1-trishydroxyphényléthane répondant à la formule 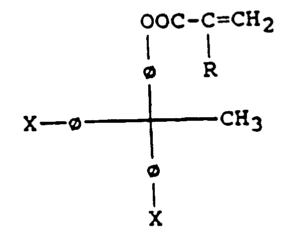 dans laquelle
φ représente un groupe phénylène
X représente un groupe -OH ou 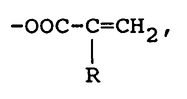 et
R reprêsente H, un groupe alkyle ou aryle.

2. Esters acryliques suivant la revendication 1, dans lesquels R représente un groupe alkyle en C₁ à C₆ ou aryle en C₆ à C₁₀.

3. Esters acryliques suivant la revendication 1, dans lesquels chaque groupe X représente un groupe

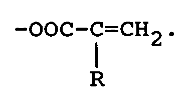

4. Composition qui comprend, en mélange
(a) un ester mono-, di- ou tri-acrylique de 1,1,1-trishydroxyphényléthane répondant à la formule 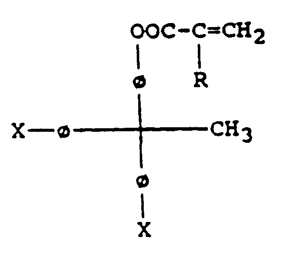 dans laquelle
φ représente un groupe phénylène
X représente un groupe -OH ou 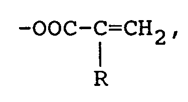 et
R représente H, un groupe alkyle ou aryle,
(b) un ou plusieurs composés choisi dans le groupe consistant en initiateurs de polymérisation par radicaux libres et agents de réticulation.

5. Article qui comprend la composition suivant la revendication 4 placée sur un substrat.

6. Composition suivant la revendication 4, comprenant un initiateur de polymérisation par radicaux libres choisi dans le groupe consistant en un composé azoïque, un peroxyde, des dérivés de quinoxaline, des dérivés de polycétaldonyle, des composés à fonction alpha-carbonyle, des éthers d'acyloïne, des dimères de triarylimidazolyle, des acyloïnes aromatiques à substituants hydrocarbonés en position alpha, des quinones polycycliques et des s-triazines.

7. Composition suivant la revendication 4, comprenant un agent de réticulation consistant en un composé de formule générale
R'O-CH₂-A-CH₂-OR'
dans laquelle A représente un groupe hydrocarboné aromatique monomérique possédant un ou plusieurs noyaux condensés ou non condensés, séparés ou non séparés, et chaque motif R' représente indépendamment H, un groupe alkyle en C₁ à C₆, cycloalkyle en C₃ à C₆, aryle ou arylalkyle.

8. Composition suivant la revendication 4, dans laquelle R représente un groupe alkyle en C₁ à C₆ ou aryle en C₆ à C₁₀.

9. Composition suivant la revendication 4, dans laquelle chaque groupe X représente un groupe

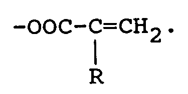

10. Polymère ou copolymère de formule 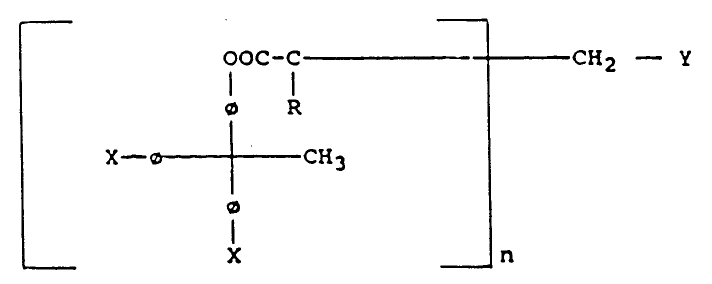 dans laquelle
φ représente un groupe phénylène
X représente un groupe -OH, 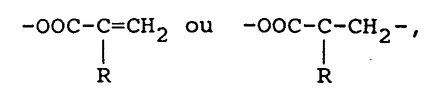 R représente H, un groupe alkyle ou aryle,
Y est choisi dans le groupe consistant en un résidu d'un composé de formule 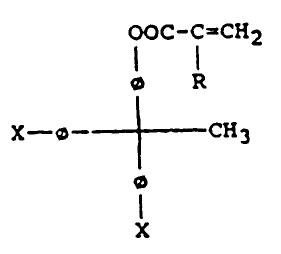 et le résidu d'un composé à insaturation éthylénique ; et n est choisi de manière à obtenir un polymère ayant un poids moléculaire compris dans l'intervalle de 200 000 à 10 000 000.

11. Polymère suivant la revendication 10, dans lequel R représente un groupe alkyle en C₁ à C₆ ou aryle en C₆ à C₁₀.

12. Polymère suivant la revendication 10, dans lequel chaque groupe X représente un groupe

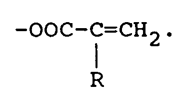

13. Polymère suivant la revendication 10, dans lequel Y représente un résidu d'un composé de formule

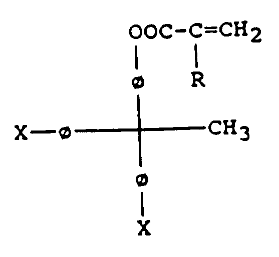

14. Polymère suivant la revendication 13, dans lequel R représente un groupe alkyle en C₁ à C₆ ou aryle en C₆ à C₁₀.

15. Polymère suivant la revendication 13, dans lequel chaque groupe X représente un groupe

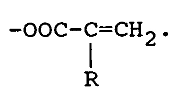

16. Copolymère suivant la revendication 10, dans lequel Y est choisi dans le groupe consistant en résidus d'acétoxystyrène, d'hydroxystyrène, d'anhydride maléique, de maléimides, de styrène, d'acrylates et de méthacrylates.

17. Composition comprenant le polymère ou copolymère suivant la revendication 10 en mélange avec un agent de réticulation.

18. Composition suivant la revendication 17, dans laquelle l'agent de réticulation consiste en un composé de formule
R'O-CH₂-A-CH₂-OR'
dans laquelle A représente un groupe hydrocarboné aromatique monomérique ayant un ou plusieurs noyaux condensés ou non condensés, séparés ou non séparés, et chaque motif R' représente indépendamment H, un groupe alkyle en C₁ à C₆, cycloalkyle en C₃ à C₆, aryle ou arylalkyle.

19. Article qui comprend la composition suivant la revendication 17 placée sur un substrat.

20. Procédé de production d'esters mono-, di- et tri-acryliques de 1,1,1-trishydroxyphényléthane répondant à la formule 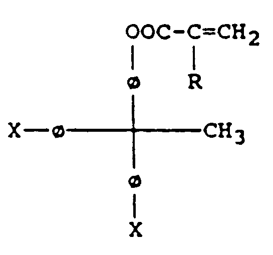 dans laquelle
φ représente un groupe phénylène,
X représente un groupe -OH ou 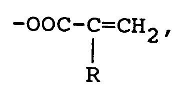 et
R représente H, un groupe alkyle ou aryle, qui comprend la réaction d'environ un équivalent de 1,1,1-tris(4'-hydroxyphényl)éthane avec environ 1 à environ 3 équivalents d'un agent d'acrylation en une quantité suffisante, pendant un temps suffisant et à une température suffisante pour produire un ester acrylique de 1,1,1-tris-hydroxyphényléthane.

21. Procédé suivant la revendication 20, dans lequel R représente un groupe alkyle en C₁ à C₆ ou aryle en C₆ à C₁₀.

22. Procédé suivant la revendication 21, dans lequel chaque groupe X représente un groupe

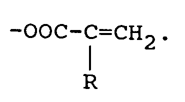

23. Procédé suivant la revendication 21, dans lequel l'agent d'acrylation est le chlorure d'acryloyle.

24. Procédé de production d'un polymère ou copolymère de formule 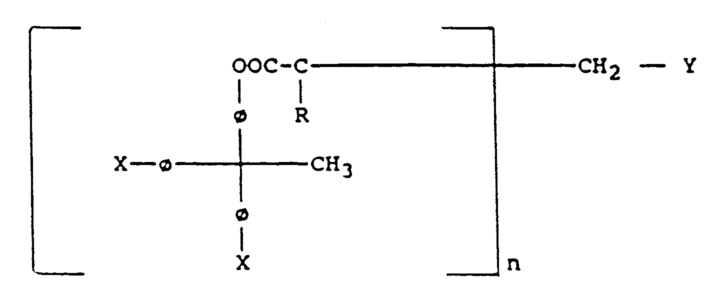 qui comprend la polymérisation par radicaux libres d'un composé répondant à la formule 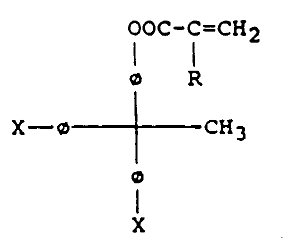 avec un composé Y qui est choisi dans le groupe consistant en un résidu d'un composé de formule 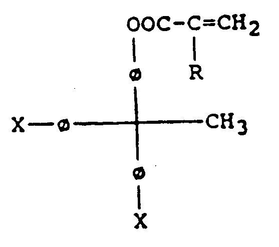 et le résidu d'un composé à insaturation éthylénique ;
φ représentant un groupe phénylène,
X représentant un groupe -OH, 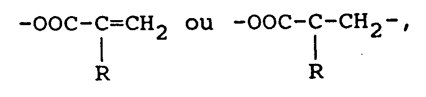 et
R représentant H, un groupe alkyle ou aryle ; et
n étant choisi de manière à obtenir un polymère ayant un poids moléculaire compris dans l'intervalle de 200 000 à 10 000 000.

25. Procédé suivant la revendication 24, qui est mis en oeuvre avec un initiateur de polymérisation par radicaux libres choisi dans le groupe consistant en un composé azoïque, un peroxyde, des dérivés de quinoxaline, des dérivés de polycétaldonyle, des composés à fonction alphacarbonyle, des éthers d'acyloïne, des dimères de triarylimidazolyle, des acyloïnes aromatiques à substituants hydrocarbonés en position alpha, des quinones polycycliques et des s-triazines.
